# EUROPÄISCHE PATENTSCHRIFT

(11) **EP 3 295 963 B1**
(45) Veröffentlichungstag und Bekanntmachung des Hinweises auf die Patenterteilung: **15.05.2019**
(21) Anmeldenummer: 17189759.8
(22) Anmeldetag: 07.09.2017
(51) Int. Cl.: A61L 2/07, B01J 3/03, B01J 3/00

(54) **AUTOKLAV MIT SICHERHEITSVERRIEGELUNGSMECHANISMUS**
AUTOCLAVE WITH SECURITY LOCK DEVICE
AUTOCLAVE AVEC SYSTEME DE VERROU SECURE

(30) Priorität: 07.09.2016 DE 102016116764
(43) Veröffentlichungstag der Anmeldung: 21.03.2018
(73) Patentinhaber: Latoschinski, Jürgen, 64850 Schaafheim (DE)
(72) Erfinder: Latoschinski, Jürgen, 64850 Schaafheim (DE)
(74) Vertreter: Habermann Intellectual Property Partnerschaft von Patentanwälten mbB

(56) Entgegenhaltungen:
- WO-A1-92/01479
- DE-U- 6 752 696
- DE-U1- 29 515 342
- GB-A- 2 074 872
- GB-A- 2 304 799
- US-A- 2 834 504
- US-A- 5 146 713
- "FINN-AQUA ? PHARMACEUTICAL GMP STEAM STERILIZER", , 5. Januar 2004 (2004-01-05), XP055211438, Gefunden im Internet: URL:http://www.equipnet.com/mp_data/media/ 2009711733_180559_1.pdf [gefunden am 2015-09-04]

## Beschreibung

Die vorliegende Erfindung betrifft einen Autoklav umfassend einen Autoklavkörper, eine Autoklavtür, ein Prüfventil zur Prüfung eines Drucks innerhalb des Autoklavs und einen Sicherheitsverriegelungsmechanismus, wobei der Sicherheitsverriegelungsmechanismus eine Bedieneinrichtung aufweist, mit welcher ein Öffnungsmodus, ein Prüfmodus und ein Verschlussmodus einstellbar sind, wobei die Autoklavtür mit einem Verriegelungsbolzen verbunden ist, mit dem der Sicherheitsverriegelungsmechanismus in eine Wirkverbindung treten kann, sodass die Autoklavtür durch den Verriegelungsbolzen gesperrt wird, wobei in dem Sicherheitsverriegelungsmechanismus ein Schaft drehbar festgelegt ist, der in einer Verriegelungsstellung mit dem Verriegelungsbolzen in eine Wirkverbindung tritt, um den Verriegelungsbolzen zu sperren, sodass die Autoklavtür gesperrt wird, und wobei der Sicherheitsverriegelungsmechanismus so eingerichtet ist, dass er in einer Verschlussstellung der Autoklavtür in dem Prüfmodus und in dem Verschlussmodus die Autoklavtür sperrt, sodass die Autoklavtür nicht geöffnet werden kann, und in dem Öffnungsmodus die Autoklavtür freigibt, sodass die Autoklavtür geöffnet werden kann, und dass er in dem Verschlussmodus das Prüfventil schließt und in dem Prüfmodus das Prüfventil öffnet, sodass der Druck innerhalb des Autoklavs geprüft werden kann, und dass der Öffnungsmodus aus dem Verschlussmodus nur unter vorheriger Einnahme des Prüfmodus erreichbar ist.

Aus dem Stand der Technik sind Autoklaven hinlänglich bekannt. Bei einem Autoklaven handelt es sich um einen druckdicht verschließbaren Behälter. Ein Material, das sich innerhalb des Behälters befindet, kann beispielsweise thermisch behandelt werden. Autoklaven können auf unterschiedliche Weise verriegelt werden, beispielsweise mittels eines Bajonettverschlusses oder eines Schaftverschlusses.

In GB 2 074 872 A ist ein Autoklav beschrieben, der eine ventilgesteuerte Türverriegelung aufweist. In US 5,146,713 ist ein Autoklav beschrieben, dessen Autoklavtür mit einem hydraulischen Mechanismus geöffnet werden kann.

Aus DE 295 15 342 U1 ist ein Sterilisierungsautoklav der eingangs genannten Gattung bekannt, dessen Autoklavtür mit einem verschiebbaren und verschwenkbaren Riegel geöffnet und verschlossen werden kann. Eine Kammer des Autoklaven ist über eine Messleitung mit einem Manometer verbunden, an welchem sich der Druck in der Kammer ablesen lässt. Zudem kann der Druck mit einem Drucksensor überwacht werden.

Soll ein unter Überdruck stehender Autoklav geöffnet werden, so ist es hierzu notwendig, zunächst den Druck innerhalb des Autoklaven in ausreichendem Maße abzusenken. Der Benutzer kann hierzu ein Druckventil des Autoklavs verwenden. Wird der Autoklav geöffnet, obwohl innerhalb des Autoklavs noch ein zu starker Druck herrscht, so kann es durch eine plötzliche Öffnung der Autoklavtür oder durch eine zu schnelle Gasentweichung aus dem Autoklaven zu Verletzungen des Bedienpersonals kommen. Deshalb ist es von herausragender Bedeutung, dass eine Überprüfung des Drucks innerhalb eines Autoklavs stets erfolgt, bevor dieser geöffnet wird. Trotzdem vergisst es das Bedienpersonal von Autoklaven zuweilen, eine entsprechende Überprüfung durchzuführen, wodurch es gelegentlich zu Verletzungen kommt. Eine weitere Problematik besteht darin, dass am Autoklaven vorgesehen Druckmesseinrichtungen wie beispielsweise Manometer nach einiger Zeit Fehlfunktionen aufweisen können. Deshalb kann nicht sichergestellt werden, dass eine Überprüfung des Drucks auf diesem Weg ein richtiges Ergebnis liefert.

Der vorliegenden Erfindung liegt deshalb die Aufgabe zugrunde, einen Autoklaven bereitzustellen, welcher die Sicherheit des Bedienpersonals bei der Öffnung des Autoklavs verbessert.

Die Aufgabe wird gelöst durch den eingangs beschriebenen Autoklaven mit den kennzeichnenden Merkmalen des Anspruchs 1.

Unter einem Autoklaven ist ein gasdicht verschließbarer Druckbehälter zu verstehen. Der erfindungsgemäße Autoklav verfügt über drei unterschiedliche Modi. Befindet sich der Autoklav in dem Verschlussmodus, so ist der Autoklav druckdicht verschlossen. Die Autoklavtür ist gesperrt, sodass sie sich nicht öffnen lässt. Das Sicherheitsventil ist in dem Verschlussmodus geschlossen. Möchte ein Benutzer den Autoklav öffnen, so muss er den Sicherheitsverriegelungsmechanismus in den Öffnungsmodus verbringen. Hierzu muss zunächst der Prüfmodus durchlaufen werden. In dem Prüfmodus ist die Autoklavtür gesperrt und das Sicherheitsventil geöffnet. Der Benutzer kann nun überprüfen, ob im Inneren des Autoklavs ein Überdruck herrscht. Durch eine Öffnung des Prüfventils kann ein im Autoklav befindliches, unter Überdruck stehendes Gas aus dem Autoklav ausströmen, wodurch gemäß einer möglichen Ausführungsform der Erfindung ein hörbares Geräusch entsteht. Der Benutzer kann somit feststellen, dass im Inneren des Autoklavs ein Überdruck herrscht und ist vor einer Öffnung des Autoklavs gewarnt. Die Autoklavtür kann weiterhin nicht geöffnet werden.

Stellt der Benutzer im Prüfmodus fest, dass das Innere des Autoklavs nicht unter einem Überdruck steht, so kann er den Sicherheitsverriegelungsmechanismus in den Öffnungsmodus verbringen. Ist ein Überdruck vorhanden, so kann dieser gemäß einer möglichen Ausführungsform des Autoklav über ein oder mehrere Ablassventile des Autoklavs abgelassen werden. In dem Öffnungsmodus gibt der Sicherheitsverriegelungsmechanismus die Autoklavtür frei, sodass diese geöffnet werden kann. Da der Sicherheitsverriegelungsmodus vor dem Öffnen stets den Prüfmodus durchläuft, ergibt sich eine verbesserte Benutzersicherheit, denn es muss stets eine Prüfung durch den Benutzer stattfinden, bevor die Autoklavtür entsperrt wird.

Erfindungsgemäß kann der Sicherheitsverriegelungsmechanismus rein mechanisch realisiert sein. Er kann jedoch auch elektromechanisch realisiert sein. Ferner kann es erfindungsgemäß vorgesehen sein, dass der Sicherheitsverriegelungsmechanismus ein Computersystem aufweist und dass insbesondere die Bedieneinrichtung zumindest teilweise durch ein Computersystem realisiert ist.

Gemäß einer besonderen Ausführungsform der Erfindung ist der Sicherheitsverriegelungsmechanismus so eingerichtet, dass er in dem Öffnungsmodus das Prüfventil schließt. Da im Öffnungsmodus kein Druckunterschied zwischen dem Inneren des Autoklavkörpers und einer Umgebungsatmosphäre des Autoklavs vorliegt, ist es nicht unbedingt notwendig, dass das Prüfventil im Öffnungsmodus geöffnet ist. Es kann jedoch zweckmäßig sein, das Prüfventil beim Übergang in den Öffnungsmodus wieder zu schließen.

Die Autoklavtür ist mit einem Verriegelungsbolzen verbunden, mit dem der Sicherheitsverriegelungsmechanismus in eine Wirkverbindung treten kann, sodass die Autoklavtür durch den Verriegelungsbolzen gesperrt wird. Die Autoklavtür kann entweder unmittelbar oder indirekt mit dem Verriegelungsbolzen verbunden sein. Gemäß letzterer Ausführungsvariante kann die Autoklavtür über ein Verbindungselement mit dem Verriegelungsbolzen verbunden sein.

Erfindungsgemäß ist in dem Sicherheitsverriegelungsmechanismus ein Schaft drehbar festgelegt, der in einer Verriegelungsstellung mit dem Verriegelungsbolzen in eine Wirkverbindung tritt, um den Verriegelungsbolzen zu sperren, sodass die Autoklavtür gesperrt wird. Der Schaft ist dazu geeignet, sowohl eine Sperrung als auch eine Freigabe des Verriegelungsbolzens zu bewirken. Ferner lässt sich mit dem Schaft das Prüfventil öffnen und verschließen. Der Schaft kann erfindungsgemäß aus einem Metall gefertigt sein und einteilig oder mehrteilig ausgeführt sein.

Es ist bevorzugt vorgesehen, dass der Schaft quer zu dem Verriegelungsbolzen angeordnet ist, sodass der Schaft oder ein an dem Schaft vorgesehenes Eingriffselement durch einen Eingriff in eine Bolzenaussparung des Verriegelungsbolzens den Verriegelungsbolzen sperrt, wenn sich der Sicherheitsverriegelungsmechanismus in dem Verschlussmodus oder in dem Prüfmodus befindet und den Verriegelungsbolzen freigibt, wenn sich der Sicherheitsverriegelungsmechanismus in dem Öffnungsmodus befindet. Die Bolzenaussparung sollte ausreichend tief ausgeführt sein, um beim Eingriff des Schafts oder des Eingriffselements in die Bolzenaussparung eine Sperrung des Verriegelungsbolzens zu gewährleisten, sodass die Autoklavtür und damit auch der Verriegelungsbolzen fest verriegelt sind. Bei dem Eingriffselement kann es sich erfindungsgemäß um einen an dem Schaft vorgesehenen Zylinder- oder Kegelstift oder um ein Eingriffselement einer sonstigen geeigneten Form handeln.

Es ist ganz besonders bevorzugt, wenn der Schaft zumindest teilweise durch die Bolzenaussparung in dem Verriegelungsbolzen verläuft, wenn sich die Autoklavtür in der Verschlussstellung befindet, und dass der Schaft zumindest im Bereich der Bolzenaussparung einen Querschnitt aufweist, der den Verriegelungsbolzen sperrt, wenn sich der Sicherheitsverriegelungsmechanismus in dem Verschlussmodus oder in dem Prüfmodus befindet und dass der Schaft den Verriegelungsbolzen freigibt, wenn sich der Sicherheitsverriegelungsmechanismus in dem Öffnungsmodus befindet. Vorzugsweise greift der Schaft in dem Verriegelungsmodus formschlüssig in die Bolzenaussparung ein. Diese Ausführungsform erlaubt eine unmittelbare Sperrung des Verriegelungsbolzens durch den Schaft, wodurch eine kompakte Bauform des Sicherheitsverriegelungsmechanismus ermöglicht wird.

Vorzugsweise weist der Querschnitt des Schafts zumindest im Bereich der Bolzenaussparung eine Kreisform mit einem fehlenden Kreissegment auf. Eine Seite des Schafts weist somit zumindest bereichsweise ein fehlendes Kreissegment auf. Befindet sich der Schaft in einer Rotationsstellung, in der die Seite des Schafts mit dem fehlenden Kreissegment der Bolzenaussparung zugewandt ist, so lässt sich der Verriegelungsbolzen bewegen. Befindet sich der Schaft in einer sonstigen Rotationsstellung, wenn sich die Autoklavtür in der Verschlussstellung befindet, so wird der Verriegelungsbolzen durch den Schaft gesperrt, sodass er nicht bewegt werden kann. In diesem Zustand lässt sich die Autoklavtür nicht öffnen.

Der Schaft kann erfindungsgemäß mit einer Prüfventilsteuerung zum Öffnen und zum Schließen des Prüfventils verbunden sein. Hierbei kann es sich erfindungsgemäß um ein drehbares Steuerelement handeln. Durch eine Drehung in eine Richtung kann hierbei eine Öffnung des Prüfventils bewirkt werden. Durch eine Drehung in eine entgegengesetzte Richtung kann eine Schließung des Prüfventils bewirkt werden. Der Schaft ist vorzugsweise mit der Prüfventilsteuerung über eine Zahnradübersetzung verbunden. Hierzu kann erfindungsgemäß an dem Schaft und an dem drehbaren Steuerelement jeweils ein Zahnrad vorgesehen sein, wobei die Zahnräder miteinander in Eingriff stehen. Eine Zahnradübersetzung ist dann besonders vorteilhaft, wenn zur Sperrung und zur Freigabe der Bewegung des Verriegelungsbolzens der Schaft Winkelpositionen einnehmen muss, die nicht mit den Winkelpositionen am drehbaren Steuerelement zur Öffnung und zum Verschluss des Prüfventils übereinstimmen.

Erfindungsgemäß weist die Bedieneinrichtung einen Führungshebel und eine durch eine Scheibe mit einer Scheibennut gebildete Führungseinrichtung aufweist, wobei die Scheibennut dazu geeignet ist, den Führungshebel aufzunehmen, sodass der Führungshebel in der Scheibennut verfahren werden kann. Durch eine Verlagerung des Führungshebels in der Scheibennut kann der Schaft in eine Verschlussposition, in eine Prüfposition und in eine Öffnungsposition verlagert werden. Durch die Führungseinrichtung wird es gewährleistet, dass wahlweise in den Verschlussmodus, in den Prüfmodus oder in den Verschlussmodus gewechselt werden kann.

Vorzugweise ist der Führungshebel solchermaßen in der Scheibennut vorgesehen, dass er entlang der Scheibennut geführt werden kann, aber nicht aus dieser entnommen werden kann.

Die Scheibennut und der Führungshebel gewährleisten, dass der Verschlussmodus, der Prüfmodus und der Öffnungsmodus angenommen werden können, indem der Führungshebel in der Scheibennut bewegt wird. Die Verschlussposition des Führungshebels entspricht dem Verschlussmodus des Sicherheitsverriegelungsmechanismus, die Prüfposition des Führungshebels entspricht dem Prüfmodus des Sicherheitsverriegelungsmechanismus und die Öffnungsposition des Führungshebels entspricht dem Öffnungsmodus des Sicherheitsverriegelungsmechanismus. Die Scheibe kann erfindungsgemäß kreisförmig ausgeführt sein. Die Scheibe kann alternativ eine abweichende Form aufweisen. Die Scheibe muss nicht notwendigerweise flach ausgebildet sein.

Erfindungsgemäß ist der Schaft durch die Scheibe hindurch mit einem Verriegelungshebel zur Drehung des Schafts verbunden ist, wobei der Verriegelungshebel eine Aussparung aufweist, durch die der Führungshebel zumindest teilweise hindurchgeführt ist. Wird der Verriegelungshebel betätigt, so lässt sich dadurch der Schaft drehen. Da der Führungshebel zumindest teilweise durch den Verriegelungshebel hindurchgeführt ist, begrenzt der Führungshebel eine Bewegung des Verriegelungshebels. Dies ist dadurch bedingt, dass die Bewegungsfreiheit des Führungshebels durch die Scheibennut begrenzt wird. Somit wird durch die Form der Scheibennut die Bewegungsfreiheit des Verriegelungshebels begrenzt. Insbesondere ist es möglich, es durch eine Bewegung des Verriegelungshebels zu bewirken, dass der Sicherheitsverriegelungsmechanismus einen Öffnungsmodus, einen Prüfmodus und einen Verschlussmodus annimmt.

Gemäß einer besonderen Ausführungsform der Erfindung ist an dem Verriegelungshebel eine Rückhalteeinrichtung vorgesehen, die eine radial in Richtung einer Verschlussführung auf der Scheibennut gerichtete Kraft auf den Führungshebel ausübt. Der Führungshebel kann sich erfindungsgemäß auf einer Verschlussführung und auf einer Öffnungsführung befinden, die durch die Aussparung in der Scheibe vorgegeben werden. Zwischen der Öffnungsführung und der Verschlussführung befindet sich ein Verbindungsabschnitt. Befindet sich der Sicherheitsverriegelungsmechanismus in dem Verschlussmodus oder wird er aus dem Verschlussmodus in den Prüfmodus überführt, so befindet sich der Führungshebel auf der Verschlussführung. Die Verschlussführung umfasst die Verschlussposition des Führungshebels. Dass ein Benutzer bei einer Betätigung des Führungshebels eine Kraft ausüben muss, signalisiert ihm, dass dies ein besonderer und potenziell gefährlicher Vorgang ist. Der Benutzer wird somit dazu angeregt, sich zu vergewissern, dass im Inneren des Autoklavs kein Überdruck herrscht.

Befindet sich der Sicherheitsverriegelungsmechanismus in dem Öffnungsmodus oder wird er aus dem Öffnungsmodus in den Prüfmodus überführt, so befindet sich der Führungshebel auf der Öffnungsführung. Die Öffnungsführung umfasst die Öffnungsposition des Führungshebels. Damit ein unbeabsichtigter Wechsel des Führungshebels auf die Öffnungsführung nicht möglich ist, ist die erfindungsgemäße Rückhalteeinrichtung vorgesehen. Die Rückhalteeinrichtung kann dabei eine Feder umfassen, die an dem Verriegelungshebel und dem Führungshebel befestigt ist. Um den Führungshebel über den Verbindungsabschnitt auf die Öffnungsführung zu verbringen, muss somit eine Kraft, die die Rückhalteeinrichtung auf den Führungshebel ausübt, überwunden werden. Ein Benutzer des Autoklavs kann hierzu den Führungshebel manuell unter Überwindung dieser Kraft umlegen.

Es ist bevorzugt, wenn die Verschlussführung entlang eines Abschnitts der Scheibe konzentrisch verläuft und die eine Öffnungsführung entlang eines Abschnitts der Scheibe konzentrisch zumindest teilweise entlang der Verschlussführung verläuft, und der Verbindungsabschnitt zwischen der Verschlussführung und der Öffnungsführung angeordnet ist. Die Scheibennut gibt somit eine Führung vor, über die durch eine Betätigung des Verriegelungshebels und eine Bewegung des Führungshebels entlang der Scheibennut die Einnahme eines Öffnungsmodus, eines Prüfmodus und eines Verschlussmodus ermöglicht wird. Die Verschlussführung und die Öffnungsführung können erfindungsgemäß so angeordnet sein, dass sich die Verschlussführung näher an einem Mittelpunkt der Scheibe befindet als die Öffnungsführung. Die Verschlussführung und die Öffnungsführung können alternativ jedoch auch so angeordnet sein, dass sich die Öffnungsführung näher an dem Mittelpunkt der Scheibe befindet als die Verschlussführung.

Vorzugsweise nimmt der Sicherheitsverriegelungsmechanismus die Verschlussposition ein, wenn sich der Führungshebel an einem Ende der inneren Führung befindet. Er nimmt besonders bevorzugt die Prüfposition ein, wenn sich der Führungshebel auf dem Verbindungsabschnitt befindet und er nimmt die Verriegelungsposition vorzugweise dann ein, wenn sich der Führungshebel an einem Ende der äußeren Führung befindet. Durch die erfindungsgemäße Ausgestaltung der Scheibennut ist es gewährleistet, dass das Prüfventil nur in dem Prüfmodus geöffnet ist. Wird der Verriegelungshebel so betätigt, sodass der Führungshebel entlang der Verschlussführung beziehungsweise entlang der Öffnungsführung geführt wird, so wird das Prüfventil geschlossen.

Vorteilhafterweise weist die Öffnungsführung eine größere Länge als die Verschlussführung auf. Befindet sich der Führungshebel auf der Öffnungsführung, so kann der Verriegelungshebel folglich weiter bewegt werden, als wenn sich der Führungshebel auf der Verschlussführung befindet. Der Verriegelungshebel kann folglich soweit bewegt werden, dass eine Freigabe des Verriegelungsbolzens durch den Sicherheitsverriegelungsmechanismus erfolgt. Die Autoklavtür kann anschließend geöffnet werden.

Gemäß einer weiteren Ausführungsform der Erfindung ist die Autoklavtür als eine Schiebetür ausgeführt. Diese lässt sich vor die Öffnung des Autoklavkörpers verbringen, um den Autoklav druckdicht zu verschließen. Um den Autoklav zu öffnen, wird die Autoklavtür seitlich verschoben. Hierdurch wird Platz vor dem Autoklaven eingespart, da für den Öffnungswinkel einer Schwenktür kein zusätzlicher Raum vorgesehen werden muss.

Es ist besonders vorteilhaft, wenn das Prüfventil mit dem Autoklavkörper über eine Druckleitung verbunden ist. Durch die Druckleitung kann ein unter Druck stehendes Gas zu dem Prüfventil geleitet werden. Das Prüfventil kann sich dabei an einer beliebigen Stelle der Druckleitung befinden. Aus einem Ende der Druckleitung kann ein Gas aus dem Autoklavkörper austreten, wenn das Prüfventil geöffnet ist. Dabei entsteht ein hörbarer Pfeifton, der es einem Benutzer des Autoklavs erlaubt, einen Überdruck im Autoklaven festzustellen.

Weitere vorteilhafte Ausgestaltungsformen der Erfindung sind in den Zeichnungen aufgeführt. Dabei zeigt:
Fig. 1 eine schematische Darstellung eines Autoklavs in einer perspektivischen Ansicht,
Fig. 2 eine schematische Darstellung einer Scheibe mit einer Scheibennut in einer Draufsicht,
Fig. 3 eine schematische Darstellung der Bedieneinrichtung des Sicherheitsverriegelungsmechanismus in einer Draufsicht, wenn der Autoklav sich im Öffnungsmodus befindet,
Fig. 4 eine schematische Darstellung der Bedieneinrichtung des Sicherheitsverriegelungsmechanismus in einer Draufsicht, wenn der Autoklav sich im Schließmodus befindet,
Fig. 5 eine schematische Darstellung der Bedieneinrichtung des Sicherheitsverriegelungsmechanismus in einer Draufsicht, wenn der Autoklav sich im Prüfmodus befindet und
Fig. 6 eine schematische Darstellung des Autoklavs, des Schafts und eines Verriegelungsbolzens in einer Schnittansicht.

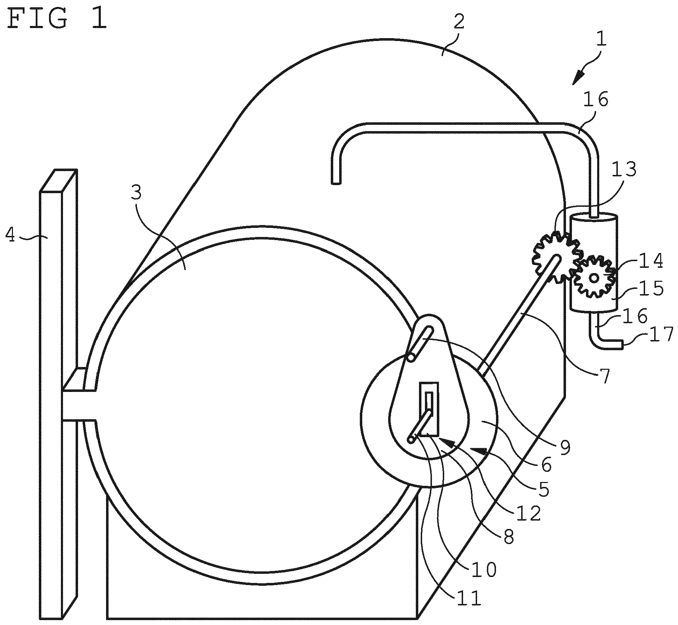

Fig. 1 zeigt eine schematische Darstellung eines Autoklavs 1 in einer perspektivischen Ansicht. Der Autoklav verfügt über einen Autoklavkörper 2, vor dem eine Autoklavtür 3 verschiebbar angebracht ist. Die Autoklavtür 3 ist so ausgeführt, dass sie das Innere des Autoklavkörpers 2 druckfest abdichtet, wenn sich die Autoklavtür 3 vor einer Öffnung des Autoklavkörpers 2 befindet. Die Autoklavtür 2 kann entlang einer Schiene 4 verfahren werden, um den Autoklav 1 wahlweise zu öffnen oder zu schließen.

An dem Autoklav ist ein Sicherheitsverriegelungsmechanismus 5 vorgesehen. Der Sicherheitsverriegelungsmechanismus 5 umfasst eine Scheibe 6, die mit dem Autoklaven 1 verbunden ist. Die Scheibe ist nicht beweglich. Ein Schaft 7 ist durch die Scheibe 6 hindurchgeführt und mit einem Verriegelungshebel 8 verbunden. An dem Verriegelungshebel 8 ist ein Bedienelement 9 vorgesehen, um eine Position des Verriegelungshebels 8 ändern zu können. Durch eine Änderung der Position des Verriegelungshebels 8 kann der Sicherheitsverriegelungsmechanismus 5 wahlweise in einen Öffnungsmodus, in einen Prüfmodus oder einen Schließmodus verbracht werden. An dem Verriegelungshebel 8 ist eine Rückhalteverkleidung 10 vorgesehen, in der sich ein eine Rückhaltevorrichtung (nicht gezeigt) befindet.

Aus der Rückhalteverkleidung 10 tritt ein Führungshebel 11 hervor. Der Führungshebel 11 muss von einem Benutzer betätigt werden, um den Sicherheitsverriegelungsmechanismus 5 aus dem Prüfmodus in den Öffnungsmodus zu versetzen. Dafür muss eine Kraft überwunden werden, die von der Rückhaltevorrichtung auf den Führungshebel 11 ausgeübt wird. Der Führungshebel 11 bildet zusammen mit dem Verriegelungshebel 8 eine Bedieneinrichtung 12 des Sicherheitsverriegelungsmechanismus 5.

Der Schaft 7 ist mit einem ersten Zahnrad 13 verbunden. Das erste Zahnrad 13 ist an einem zweiten Zahnrad 14 so angeordnet, dass das erste Zahnrad 13 in das zweite Zahnrad 14 eingreift. Das zweite Zahnrad 14 ist an einem Prüfventil 15 drehbar befestigt. Durch eine Drehung des zweiten Zahnrads 14 wird das Prüfventil 15 geöffnet beziehungsweise geschlossen. Eine Druckleitung 16 führt aus dem Autoklavkörper 2 heraus, wobei an der Druckleitung das Prüfventil 15 vorgesehen ist. Ein unter Überdruck stehendes Gas aus dem Inneren des Autoklavskörpers 2 kann aus einem Ende der Druckleitung 17 austreten, wenn das Prüfventil 15 geöffnet ist. Dabei entsteht ein hörbares Pfeifgeräusch.

Der Sicherheitsverriegelungsmechanismus 5 ist so eingerichtet, dass er in einer Verschlussstellung der Autoklavtür 3 in dem Öffnungsmodus eine Bewegung der Autoklavtür 3 freigibt und in dem Prüfmodus und in dem Verschlussmodus eine Bewegung der Autoklavtür 3 sperrt, sodass die Autoklavtür 3 nicht geöffnet werden kann. Er ist außerdem so eingerichtet, dass er in dem Öffnungsmodus und in dem Verschlussmodus das Prüfventil 15 schließt und in dem Prüfmodus das Prüfventil 15 öffnet, sodass ein Druck innerhalb des Autoklavs 1 geprüft werden kann, und dass der Öffnungsmodus aus dem Verschlussmodus nur unter Durchlauf des Prüfmodus erreichbar ist.

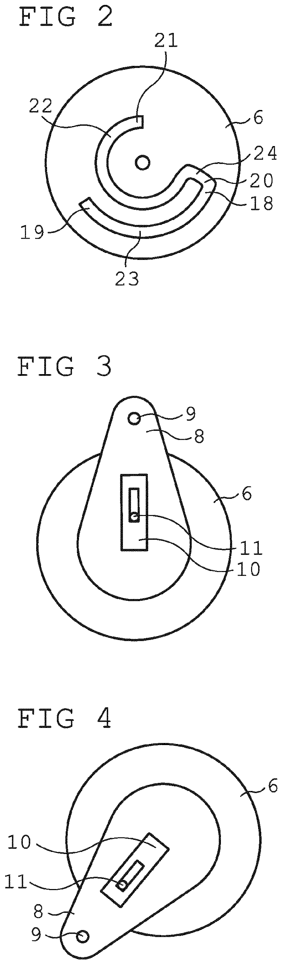

Fig. 2 zeigt eine schematische Darstellung der Scheibe 6 mit einer Scheibennut 18 in einer Draufsicht. Die Scheibennut 18 ist dazu geeignet, den Führungshebel 11 aufzunehmen. Der Führungshebel 11 kann entlang der Scheibennut 18 verfahren werden. Die Scheibennut 18 der Scheibe 6 begrenzt die Bewegungsfreiheit des Führungshebels 17 und damit auch die Bewegungsfreiheit des Verriegelungshebels 8. Innerhalb der Scheibennut 18 kann der Führungshebel 11 an eine Verschlussposition 19, an eine Prüfposition 20 und an eine Öffnungsposition 21 verbracht werden. Die Verschlussposition 19 entspricht dem Verschlussmodus des Sicherheitsverriegelungsmechanismus 5, die Prüfposition 20 entspricht dem Prüfmodus des Sicherheitsverriegelungsmechanismus 5 und die Öffnungsposition 21 entspricht dem Öffnungsmodus des Sicherheitsverriegelungsmechanismus 5. Die Scheibennut 18 ist untergliedert in eine Öffnungsführung 22, eine Verschlussführung 23 und einen Verbindungsabschnitt 24, wobei der Verbindungsabschnitt 24 die Öffnungsführung 22 und die Verschlussführung 23 verbindet.

Fig. 3 zeigt eine schematische Darstellung der Bedieneinrichtung 12 des Sicherheitsverriegelungsmechanismus 5 in einer Draufsicht, wenn der Autoklav 1 sich im Öffnungsmodus befindet. Der Führungshebel 11 befindet sich in der Öffnungsposition 21 und der Verriegelungshebel 8 ist nach oben ausgerichtet. Durch den Schaft 7 ist das Prüfventil 15 geschlossen und die Autoklavtür 3 ist freigegeben, sodass diese geöffnet werden kann.

Fig. 4 zeigt eine schematische Darstellung der Bedieneinrichtung 12 des Sicherheitsverriegelungsmechanismus 5 in einer Draufsicht, wenn der Autoklav 1 sich im Öffnungsmodus befindet. Der Führungshebel 11 befindet sich in der Verschlussposition 19 und der Verriegelungshebel 8 ist nach links unten ausgerichtet. Durch den Schaft 7 ist das Prüfventil 15 geschlossen und die Autoklavtür 3 ist gesperrt, sodass diese nicht geöffnet werden kann.

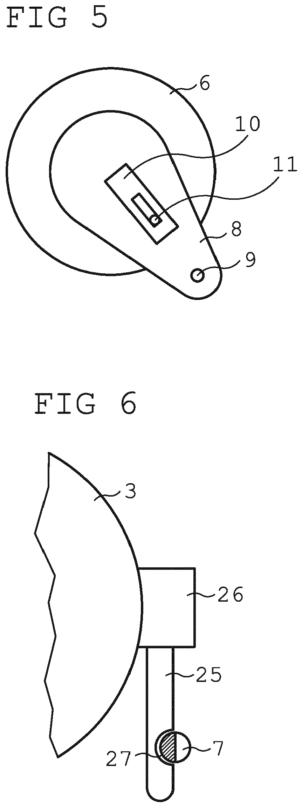

Fig. 5 zeigt eine schematische Darstellung der Bedieneinrichtung 12 des Sicherheitsverriegelungsmechanismus 5 in einer Draufsicht, wenn der Autoklav 1 sich im Prüfmodus befindet. Der Führungshebel 11 befindet sich in der Prüfposition 20 und der Verriegelungshebel 8 ist nach rechts unten ausgerichtet. Durch den Schaft 7 ist das Prüfventil 15 geöffnet und die Autoklavtür 3 ist gesperrt, sodass diese nicht geöffnet werden kann. Durch eine Betätigung des Verriegelungshebels 8 lässt sich an der Bedieneinrichtung 12 der Verschlussmodus einstellen. Soll hingegen der Öffnungsmodus an der Bedieneinrichtung 12 eingestellt werden, so muss zunächst der Führungshebel 11 gegen eine Rückhaltekraft verstellt und gehalten werden, wobei anschließend durch eine Betätigung des Verriegelungshebels 8 in den Öffnungsmodus gewechselt werden kann. Durch die Rückstellung des Führungshebels 11 bestätigt ein Benutzer, dass er eine Öffnung des Autoklavs 1 wünscht. Die dabei zu überwindende Kraft kann den Benutzer darauf hinweisen, dass vor einer Öffnung des Autoklavs 1 dessen Innendruck überprüft werden sollte.

Fig. 6 zeigt eine schematische Darstellung der Autoklavtür 3, des Schafts 7 und eines Verriegelungsbolzens 25 in einer Schnittansicht. Der Verriegelungsbolzen 25 ist mit der Autoklavtür 3 über ein Verbindungselement 26 verbunden. Die Autoklavtür 3 schließt den in Fig. 6 nicht vollständig gezeigten Autoklaven 1. Dabei befindet sich eine Bolzenaussparung 27 im Verriegelungsbolzen 25 auf Höhe des Schafts 7. In der Darstellung ist der Schaft 7 solchermaßen ausgerichtet, dass der Verriegelungsbolzen 25 beweglich ist. Durch eine Drehung des Schafts 7 kann dieser solchermaßen ausgerichtet werden, dass er in die Bolzenaussparung 27 eingreift, sodass der Verriegelungsbolzen 25 gesperrt wird.

### BEZUGSZEICHENLISTE

- 1.: Autoklav
- 2.: Autoklavkörper
- 3.: Autoklavtür
- 4.: Schiene
- 5.: Sicherheitsverriegelungsmechanismus
- 6.: Scheibe
- 7.: Schaft
- 8.: Verriegelungshebel
- 9.: Bedienelement
- 10.: Rückhalteverkleidung
- 11.: Führungshebel
- 12.: Bedieneinrichtung
- 13.: Erstes Zahnrad
- 14.: Zweites Zahnrad
- 15.: Prüfventil
- 16.: Druckleitung
- 17.: Ende der Druckleitung
- 18.: Scheibennut
- 19.: Verschlussposition
- 20.: Prüfposition
- 21.: Öffnungsposition
- 22.: Öffnungsführung
- 23.: Verschlussführung
- 24.: Verbindungsabschnitt
- 25.: Verriegelungsbolzen
- 26.: Verbindungselement
- 27.: Bolzenaussparung

## Patentansprüche

1. Autoklav (1) umfassend einen Autoklavkörper (2), eine Autoklavtür (3), ein Prüfventil (15) zur Prüfung eines Drucks innerhalb des Autoklavs (1) und einen Sicherheitsverriegelungsmechanismus (5), wobei der Sicherheitsverriegelungsmechanismus (5) eine Bedieneinrichtung (12) aufweist, mit welcher ein Öffnungsmodus, ein Prüfmodus und ein Verschlussmodus einstellbar sind, wobei die Autoklavtür (3) mit einem Verriegelungsbolzen (25) verbunden ist, mit dem der Sicherheitsverriegelungsmechanismus (5) in eine Wirkverbindung treten kann, sodass die Autoklavtür (3) durch den Verriegelungsbolzen (25) gesperrt wird, wobei in dem Sicherheitsverriegelungsmechanismus (5) ein Schaft (7) drehbar festgelegt ist, der in einer Verriegelungsstellung mit dem Verriegelungsbolzen (25) in eine Wirkverbindung tritt, um den Verriegelungsbolzen (25) zu sperren, sodass die Autoklavtür (3) gesperrt wird, und wobei der Sicherheitsverriegelungsmechanismus (5) so eingerichtet ist, dass er in einer Verschlussstellung der Autoklavtür (3) in dem Prüfmodus und in dem Verschlussmodus die Autoklavtür (25) sperrt, sodass die Autoklavtür (25) nicht geöffnet werden kann, und in dem Öffnungsmodus die Autoklavtür (25) freigibt, sodass die Autoklavtür (3) geöffnet werden kann, und dass er in dem Verschlussmodus das Prüfventil (15) schließt und in dem Prüfmodus das Prüfventil (15) öffnet, sodass der Druck innerhalb des Autoklavs (1) geprüft werden kann, und dass der Öffnungsmodus aus dem Verschlussmodus nur unter vorheriger Einnahme des Prüfmodus erreichbar ist, **dadurch gekennzeichnet, dass** die Bedieneinrichtung (11) einen Führungshebel (11) und eine durch eine Scheibe (6) mit einer Scheibennut (18) gebildete Führungseinrichtung aufweist, wobei die Scheibennut dazu geeignet ist, den Führungshebel (11) aufzunehmen, sodass der Führungshebel (11) in der Scheibennut (18) verfahren werden kann, dass der Schaft (7) durch die Scheibe (6) hindurch mit einem Verriegelungshebel (8) zur Drehung des Schafts (7) verbunden ist, wobei der Verriegelungshebel (8) eine Aussparung aufweist, durch die der Führungshebel (11) zumindest teilweise hindurchgeführt ist, und dass der Schaft (7) mit einer Prüfventilsteuerung zum Öffnen und Schließen des Prüfventils verbunden ist.

2. Autoklav nach Anspruch 1, **dadurch gekennzeichnet, dass** der Sicherheitsverriegelungsmechanismus (5) so eingerichtet ist, dass er in dem Öffnungsmodus das Prüfventil (15) schließt.

3. Autoklav (1) nach Anspruch 1 oder 2, **dadurch gekennzeichnet, dass** der Schaft (7) quer zu dem Verriegelungsbolzen (25) angeordnet ist, sodass der Schaft (7) oder ein an dem Schaft (7) vorgesehenes Eingriffselement durch einen Eingriff in eine Bolzenaussparung (27) des Verriegelungsbolzens (25) den Verriegelungsbolzen (25) sperrt, wenn sich der Sicherheitsverriegelungsmechanismus (5) in dem Verschlussmodus oder in dem Prüfmodus befindet und den Verriegelungsbolzens (25) freigibt, wenn sich der Sicherheitsverriegelungsmechanismus (5) in dem Öffnungsmodus befindet.

4. Autoklav (1) nach Anspruch 3, **dadurch gekennzeichnet, dass** der Schaft (7) zumindest teilweise durch die Bolzenaussparung (27) in dem Verriegelungsbolzen (25) verläuft, wenn sich die Autoklavtür (3) in der Verschlussstellung befindet, und dass der Schaft (7) zumindest im Bereich der Bolzenaussparung (27) einen Querschnitt aufweist, der den Verriegelungsbolzen (25) sperrt, wenn sich der Sicherheitsverriegelungsmechanismus (5) in dem Verschlussmodus oder in dem Prüfmodus befindet und den Verriegelungsbolzen (25) freigibt, wenn sich der Sicherheitsverriegelungsmechanismus (5) in dem Öffnungsmodus befindet.

5. Autoklav (1) nach Anspruch 4, **dadurch gekennzeichnet, dass** der Querschnitt des Schafts (7) zumindest im Bereich der Bolzenaussparung (27) eine Kreisform mit einem fehlenden Kreissegment aufweist.

6. Autoklav (1) nach einem der vorhergehenden Ansprüche, **dadurch gekennzeichnet, dass** der Schaft (7) mit der Prüfventilsteuerung über eine Zahnradübersetzung verbunden ist.

7. Autoklav (1) nach einem der vorhergehenden Ansprüche, **dadurch gekennzeichnet, dass** an dem Verriegelungshebel (8) eine Rückhalteeinrichtung vorgesehen ist, die eine radial in Richtung einer Verschlussführung (23) auf der Scheibennut (18) gerichtete Kraft auf den Führungshebel (11) ausübt.

8. Autoklav (1) nach einem der vorhergehenden Ansprüche, **dadurch gekennzeichnet, dass** die Scheibennut (18) eine Verschlussführung (23), die entlang eines Abschnitts der Scheibe (6) konzentrisch verläuft, eine Öffnungsführung (22), die entlang eines Abschnitts der Scheibe (6) konzentrisch zumindest teilweise entlang der Verschlussführung (23) verläuft, und einen Verbindungsabschnitt (24) zwischen der Verschlussführung (23) und der Öffnungsführung (22) aufweist.

9. Autoklav (1) nach Anspruch 8, **dadurch gekennzeichnet, dass** der Sicherheitsverriegelungsmechanismus (5) den Verschlussmodus einnimmt, wenn sich der Führungshebel (11) an einem Ende der Öffnungsführung (22) befindet, dass der Sicherheitsverriegelungsmechanismus (5) den Prüfmodus einnimmt, wenn sich der Führungshebel (11) auf dem Verbindungsabschnitt (24) befindet und dass der Sicherheitsverriegelungsmechanismus (5) den Verriegelungsmodus einnimmt, wenn sich der Führungshebel (11) an einem Ende der Verschlussführung (23) befindet.

## Claims

1. Autoclave (1), comprising an autoclave body (2), an autoclave door (3), a test valve (15) for testing a pressure inside the autoclave (1), and a safety lock mechanism (5), wherein the safety lock mechanism (5) comprises an operating device (12), by means of which an opening mode, a test mode and a locking mode can be set, wherein the autoclave door (3) is connected with a locking bolt (25), with which the safety lock mechanism (5) can enter an operative connection, so that the autoclave door (3) is blocked by means of the locking bolt (25), wherein a shaft (7) is rotatably mounted in the safety lock mechanism (5), which shaft enters an operative connection with the locking bolt (25) in a locking position to lock the locking bolt (25), so that the autoclave door (3) is locked, and wherein the safety lock mechanism (5) is configured in such a way that it locks the autoclave door (25) in a locking position of the autoclave door (3) in the test mode and in the locking mode, so that the autoclave door (25) can not be opened, and releases the autoclave door (25) in an opening mode so that the autoclave door (3) can be opened, and that it closes the test valve (15) in the locking mode and opens the test valve (15) in the test mode, so that the pressure inside the autoclave (1) can be tested, and that the opening mode can be reached from the locking mode only upon entering the test mode before, **characterized in that** the operating device (11) comprises a guide lever (11) and a guide mechanism formed by a disk (6) with a disk groove (18), wherein the disk groove is suitable to receive the guide lever (11), so that the guide lever (11) can be displaced in the disk groove (18), **in that** the shaft (7) is connected, through the disk (6), with a locking lever (8) for rotating the shaft (7), wherein the locking lever (8) comprises a recess, through which the guide lever (11) is at least partially guided, and **in that** the shaft (7) is connected with a test valve control for the opening and closing of the test valve.

2. Autoclave according to claim 1, **characterized in that** the safety lock mechanism (5) is configured in such a way that it closes the test valve (15) in the opening mode.

3. Autoclave (1) according to claim 1 or 2, **characterized in that** the shaft (7) is arranged transversally to the locking bolt (25), so that the shaft (7) or an engagement element provided on the shaft (7) blocks the locking bolt (25) by engagement into a bolt recess (27) of the locking bolt (25) when the safety lock mechanism (5) is in the locking mode or in the test mode, and releases the locking bolt (25) when the safety lock mechanism (5) is in the opening mode.

4. Autoclave (1) according to claim 3, **characterized in that** the shaft (7) at least partially extends through the bolt recess (27) in the locking bolt (25) when the autoclave door (3) is in the locking position, and **in that** the shaft (7) in the region of the bolt recess (27) has a cross-section that blocks the locking bolt (25) when the safety lock mechanism (5) is in the locking mode or in the test mode, and releases the locking bolt (25) when the safety lock mechanism (5) is in the opening mode.

5. Autoclave (1) according to claim 4, **characterized in that** the cross-section of the shaft (7), at least in the region of the bolt recess (27), has a circular shape with a missing segment of a circle.

6. Autoclave (1) according to any of the preceding claims, **characterized in that** the shaft (7) is connected with the test valve control via a gear train.

7. Autoclave (1) according to any of the preceding claims, **characterized in that** a retaining element is provided on the locking lever (8), which element exerts a force on to the guide lever (11) directed radially in the direction of a closure guidance (23) on the disk groove (18).

8. Autoclave (1) according to any of the preceding claims, **characterized in that** the disk groove (18) comprises a closure guidance (23) which extends concentrically along a section of the disk (6), an opening guidance (22) which extends concentrically along a section of the disk (6) at least partially along the closure guidance (23), and a connection portion (24) between the closure guidance (23) and the opening guidance (22).

9. Autoclave (1) according to claim 8, **characterized in that** the safety lock mechanism (5) enters the locking mode when the guide lever (11) is located at an end of the opening guidance (22), **in that** the safety lock mechanism (5) enters the test mode when the guide lever (11) is located on the connection portion (24), and **in that** the safety lock mechanism (5) enters the blocking mode when the guide lever (11) is located on an end of the closure guidance (23).

## Revendications

1. Autoclave (1) comprenant un corps d'autoclave (2), une porte d'autoclave (3), une soupape de contrôle (15) pour le contrôle d'une pression à l'intérieur de l'autoclave (1) et un système de verrouillage de sécurité (5), le système de verrouillage de sécurité (5) comportant un dispositif de commande (12), au moyen duquel un mode ouverture, un mode contrôle et un mode fermeture peuvent être réglés, la porte d'autoclave (3) étant reliée à un boulon de verrouillage (25), avec lequel le système de verrouillage de sécurité (5) peut coopérer, de telle sorte que la porte d'autoclave (3) est bloquée par le boulon de verrouillage (25), une tige (7) étant fixée de manière rotative dans le système de verrouillage de sécurité (5), laquelle, dans une position de verrouillage, coopère avec le boulon de verrouillage (25) pour bloquer le boulon de verrouillage (25), de telle sorte que la porte d'autoclave (3) est bloquée, et le système de verrouillage de sécurité (5) étant conçu de telle manière que, dans une position de fermeture de la porte d'autoclave (3), il bloque la porte d'autoclave (25) en mode contrôle et en mode fermeture, de telle sorte que la porte d'autoclave (25) ne peut pas être ouverte, et libère la porte d'autoclave (25) en mode ouverture, de telle sorte que la porte d'autoclave (3) peut être ouverte, et que, en mode fermeture, il ferme la soupape de contrôle (15) et en mode contrôle, il ouvre la soupape de contrôle (15), de telle sorte que la pression à l'intérieur de l'autoclave (1) peut être contrôlée, et que le mode ouverture peut être atteint à partir du mode fermeture uniquement après un passage préalable par le mode contrôle, **caractérisé en ce que** le dispositif de commande (11) comporte un levier de guidage (11) et un dispositif de guidage formé par un disque (6) doté d'une rainure de disque (18), la rainure de disque étant adaptée pour recevoir le levier de guidage (11), de telle sorte que le levier de guidage (11) peut être déplacé dans la rainure de disque (18), **en ce que** la tige (7) est reliée à un levier de verrouillage (8) à travers le disque (6) pour la rotation de la tige (7), le levier de verrouillage (8) comportant un évidement, à travers lequel le levier de guidage (11) est au moins en partie guidé, et **en ce que** la tige (7) est reliée à une commande de soupape de contrôle pour ouvrir et fermer la soupape de contrôle.

2. Autoclave selon la revendication 1, **caractérisé en ce que** le système de verrouillage de sécurité (5) est conçu de telle manière que, en mode ouverture, il ferme la soupape de contrôle (15).

3. Autoclave (1) selon la revendication 1 ou 2, **caractérisé en ce que** la tige (7) est disposée transversalement au boulon de verrouillage (25), de sorte que la tige (7) ou un élément d'engagement prévu sur la tige (7) bloque le boulon de verrouillage (25) par un engagement dans un évidement de boulon (27) du boulon de verrouillage (25), quand le système de verrouillage de sécurité (5) se trouve en mode fermeture ou en mode contrôle, et libère le boulon de verrouillage (25) quand le système de verrouillage de sécurité (5) se trouve en mode ouverture.

4. Autoclave (1) selon la revendication 3, **caractérisé en ce que** la tige (7) s'étend au moins partiellement à travers l'évidement de boulon (27) du boulon de verrouillage (25), quand la porte d'autoclave (3) se trouve dans la position de fermeture, et **en ce que** la tige (7) présente, au moins dans la zone de l'évidement de boulon (27), une section transversale qui bloque le boulon de verrouillage (25), quand le système de verrouillage de sécurité (5) se trouve en mode fermeture ou en mode contrôle, et libère le boulon de verrouillage (25) quand le système de verrouillage de sécurité (5) se trouve en mode ouverture.

5. Autoclave (1) selon la revendication 4, **caractérisé en ce que** la section transversale de la tige (7) présente, au moins dans la zone de l'évidement de boulon (27), une forme circulaire avec un segment de cercle manquant.

6. Autoclave (1) selon l'une des revendications précédentes, **caractérisé en ce que** la tige (7) est reliée à la commande de soupape de contrôle par le biais d'un engrenage.

7. Autoclave (1) selon l'une des revendications précédentes, **caractérisé en ce qu'**un dispositif de retenue, qui exerce sur le levier de guidage (11) une force orientée radialement dans la direction d'un guidage de fermeture (23) sur la rainure de disque (18), est prévu sur le levier de verrouillage (8).

8. Autoclave (1) selon l'une des revendications précédentes, **caractérisé en ce que** la rainure de disque (18) comporte un guidage de fermeture (23), qui s'étend de manière concentrique le long d'une portion du disque (6), un guidage d'ouverture (22), qui s'étend de manière concentrique le long d'une portion du disque (6) au moins en partie le long du guidage de fermeture (23), et une portion de liaison (24) entre le guidage de fermeture (23) et le guidage d'ouverture (22).

9. Autoclave (1) selon la revendication 8, **caractérisé en ce que** le système de verrouillage de sécurité (5) passe en mode fermeture quand le levier de guidage (11) se trouve à une extrémité du guidage d'ouverture (22), **en ce que** le système de verrouillage de sécurité (5) passe en mode contrôle quand le levier de guidage (11) se trouve sur la portion de liaison (24) et **en ce que** le système de verrouillage de sécurité (5) passe en mode verrouillage quand le levier de guidage (11) se trouve à une extrémité du guidage de fermeture (23).
